(19) Europäisches Patentamt

European Patent Office

Office européen des brevets

(11) Publication number: **0 293 032 B1**

(12) **EUROPEAN PATENT SPECIFICATION**

(45) Date of publication of patent specification: **21.07.93**

(51) Int. Cl.5: **C01B 35/10**, C01B 33/34, C01B 33/20, B01J 29/04, B01J 20/10

(21) Application number: **88200950.9**

(22) Date of filing: **11.05.88**

(54) **Synthetic, crystalline, porous material containing oxides of silicon and boron.**

(30) Priority: **26.05.87 IT 2067087**

(43) Date of publication of application:
**30.11.88 Bulletin 88/48**

(45) Publication of the grant of the patent:
**21.07.93 Bulletin 93/29**

(84) Designated Contracting States:
**AT BE CH DE ES FR GB GR LI LU NL SE**

(56) References cited:
**EP-A- 0 059 059**
**EP-A- 0 100 115**
**FR-A- 2 573 326**
**GB-A- 2 144 727**

(73) Proprietor: **ENIRICERCHE S.p.A.**
**Corso Venezia 16**
**I-20121 Milan(IT)**

Proprietor: **ENICHEM SYNTHESIS S.p.A.**
**Via Ruggero Settimo 55**
**I-90139 Palermo(IT)**

Proprietor: **SNAMPROGETTI S.p.A.**

**Corso Venezia 16**
**I-20121 Milan(IT)**

(72) Inventor: **Bellussi, Giuseppe**
**Via Alberto Scoto 44**
**I-29100 Piacenza(IT)**
Inventor: **Perego, Giovanni**
**Piazzale S. di Santarosa 4**
**I-20156 Milan(IT)**
Inventor: **Clerici, Mario Gabriele**
**Via Europa 34**
**I-20097 San Donato Milanese Milan(IT)**
Inventor: **Giusti, Aldo**
**Via di Meassino 10**
**I-55100 Lucca(IT)**

(74) Representative: **Roggero, Sergio et al**
**Ing. Barzanò & Zanardo Milano S.p.A. Via Borgonuovo 10**
**I-20121 Milano (IT)**

Rank Xerox (UK) Business Services
(3.10/3.6/3.3.1)

**Description**

The present invention relates to synthetic, crystalline and porous materials containing Si oxide and B oxide, and to the method of their preparation.

Several synthetic materials based on Si and B oxides are known, such as the zeolite called AMS-IB, prepared in the presence of the tetrapropylammonium cation, and disclosed in BE-A-859,656.

BE-A-877,205 discloses several synthetic materials of that kind, having a crystalline porous structure: these are synthesized in the presence of cations capable of addressing the synthesis towards the desired structure, and are called BOR-A (tetramethylammonium), BOR-B (tetraethylammonium), BOR-C (tetraethylammonium or tetrapropylammonium or a cation derived from ethylenediamine) and BOR-D (tetrabutylammonium).

BE-A-896,686 discloses boralites BOR-E, having a structure intermediate between BOR-C and BOR-D (mixture of tetraethylammonium and tetrapropylammonium, or methylbutylammonium).

FR-A-2 573 326 discloses a zeolite consisting of Si- and B-oxides, which is prepared in the presence of an optically active enantiomer of the N,N-dimethyl-3-methyl-piperidinium cation.

The present Applicants have now found a synthetic zeolite containing oxides of silicon and boron, the synthesis of which is realized in the presence of piperidine or hexamethyleneimine, which can be used either as a molecular sieve, or as an ion-exchanger, or as a catalyst in several reactions among which, for exemplifying purposes, hydrocarbon cracking, oligomerization of olefins and skeleton isomerization can be mentioned.

The object of the present invention is a crystalline, porous material containing oxides of silicon and boron and having, in the non-calcined state, the following empirical formula:

$$mM_{2/n}O, pR, xM^I_2O_3, (1-x)B_2O_3, yM^{II}O_2, qH_2O$$

and having, in the calcined and anhydrous state, the following general formula:

$$mM_{2/n}O, xM^I_2O_3, (1-x)B_2O_3, yM^{II}O_2,$$

wherein :
$M$     is an $H^+$, $NH_4^+$ or a metal cation with valence n,
$M^I$     is a trivalent metal selected from Al, Cr, Fe, Ga, V or Mn, capable of replacing a portion of boron in the crystal lattice,
$M^{II}$     is either Si or mixtures thereof with such tetravalent metals as Ge, Ti or Zr, capable of replacing a portion of silicon in the crystal lattice,
$R$     is piperidine or hexamethyleneimine,
$m$     has a value comprised within the range of from 0.1 to 1.5,
$x$     has a value comprised within the range of from 0 to 0.9, preferably equal to zero or smaller than 0.5,
$y$     has a value greater than 2 and preferably comprised within the range of from 5 to 300,
$p$     has a value greater than zero and smaller than 3, and
$q$     has a value greater than zero and smaller than 5,
and having a volume of the pores larger than 0.3 cm³/g, preferably equal to, or larger than, 0.35 cm³/g.

The passage from a cationic form to a different cationic form can be carried out by means of the usual exchange processes known from the prior art.

The synthetic material in accordance with the present invention results crystalline on X-ray analysis. Such an analysis was carried out by means of a powder diffractometer equipped with an electronic impulse counting system, using Cukα radiation. For computing the values of the intensities, the heights of the peaks were measured and the relevant percentages relatively to the height of the most intense peak were computed.

The main reflections for the product dried at 120°C are characterized by the following $d$ values (wherein $d$ is the interplanar distance):

2

| d (Å = $10^{-1}$nm) | 100 I/I$_o$ |
|---|---|
| 27.1 ± 0.4 | 70-95 |
| 13.5 ± 0.3 | 80-100 |
| 12.3 ± 0.2 | 100 |
| 11.1 ± 0.2 | 40-50 |
| 9.2 ± 0.1 | 30-40 |
| 9.0 ± 0.1 | 30-40 |
| 4.45 ± 0.05 | 25-35 |
| 3.98 ± 0.05 | 25-35 |
| 3.53 ± 0.05 | 15-30 |
| 3.39 ± 0.05 | 45-55 |
| 3.30 ± 0.05 | 25-35 |

The main reflections for the product calcined at 550 °C are characterized by the following d values:

| d (Å = $10^{-1}$nm) | 100 I/I$_o$ |
|---|---|
| 12.3 ± 0.2 | 100 |
| 11.05 ± 0.2 | 65-75 |
| 8.80 ± 0.1 | 55-70 |
| 6.82 ± 0.05 | 10-20 |
| 6.22 ± 0.05 | 20-35 |
| 6.15 ± 0.05 | 20-35 |
| 5.52 ± 0.05 | 10-20 |
| 4.36 ± 0.05 | 10-20 |
| 4.08 ± 0.05 | 15-25 |
| 4.03 ± 0.05 | 15-25 |
| 3.88 ± 0.05 | 30-40 |
| 3.72 ± 0.05 | 15-25 |
| 3.40 ± 0.05 | 35-50 |

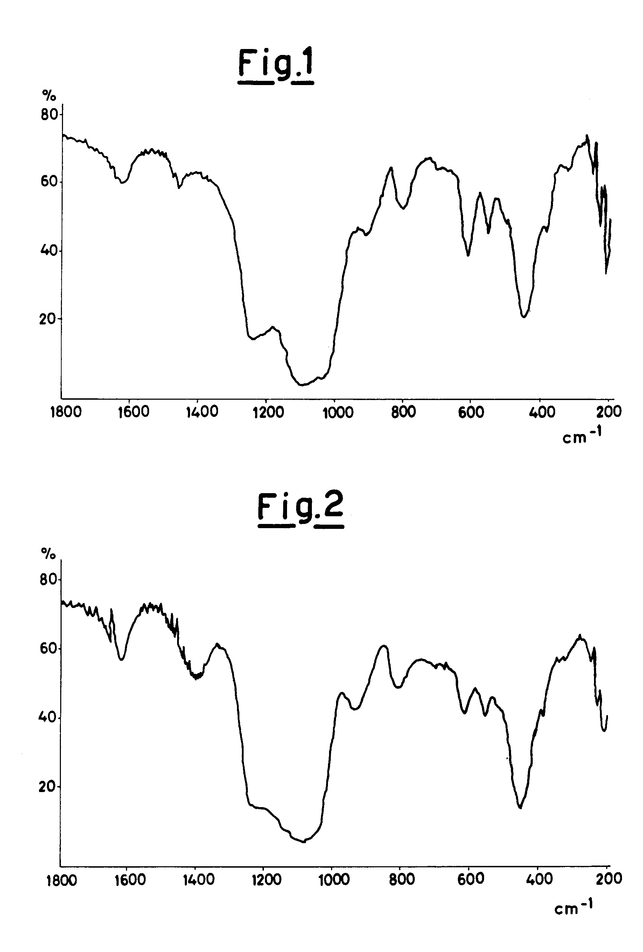

In Figure 1, the I.R. spectrum of non-calcined product, and in Figure 2 the spectrum of calcined product is shown (on the abscissa, the wave number as $cm^{-1}$, and on the ordinate the transmittance % is reported). Another aspect of the present invention is a process for hydrothermally preparing a synthetic, crystalline and porous material as defined hereinabove, comprising the step of reacting, at a temperature of from 130 °C to 250 °C, at a pH of from 8 to 13, and for a time of from 1 day to 30 days, a silicon derivative, a boron derivative and an organic nitrogenous base selected from piperidine and hexamethyleneimine, with the facultative addition of a derivative of a trivalent metal selected from Al, Cr, Fe, Ga, V and Mn and/or a derivative of a tetravalent metal selected from Ge, Ti , and Zr, and/or a sodium salt or a sodium hydroxide to which other alkali metal or alkaline earth metal salts and/or hydroxides may be added, under the conditions that:

the molar ratio $SiO_2$ to $B_2O_3$ in the reactants is from 0,5 to 10;

the molar ratio R to $SiO_2$ in the reactants is from 0,75 to 1,4;

the molar ratio $M^I_2O_3$ to $SiO_2$ in the reactants is from nil to 0,02;

the molar ratio $M^{II}O_2$ to $SiO_2$ in the reactants is from 1 to 3, and

the molar ratio Na to $SiO_2$ in the reactants is from nil to 0,5.

The molar ratio of $H_2O/SiO_2$ of the reactants is preferably comprised within the range of from 5 to 50.

The silicon derivative is selected from silica gel, silica sol, aerosil, colloidal silica, sodium silicate or an alkyl-silicate.

The boron derivative can be selected from trialkylborates, alkaline borates, ammonium borate and boric acid.

The derivative of the trivalent metal and/or of the tetravalent metal used can be selected from the corresponding salts.

Some examples are given now for the purpose of better illustrating the invention, which however should not be construed as being in any way limited by them, or to them.

3

## Examples

The synthesis examples which follow were carried out according to the hereunder reported procedure.

To a pyrex glass vessel equipped with a stirrer, the required amount of demineralized water is charged. In this water, firstly sodium hydroxide, when envisaged, and then the amine are dissolved with stirring.

The alkaline solution is heated, with stirring, to 30-50°C, and to it boric acid is then added; the temperature is then adjusted at a value comprised within the range of from 50 to 80°C, until the acid is completely dissolved. To the so-obtained, generally clear, solution, the source of silica is added in such a way that the addition is complete within 1-2 hours. The mixture is then maintained stirred at 70°C for a further hour, and is then cooled down to room temperature; after recording its pH, the mixture is charged to a stainless-steel autoclave which is fastened on a swinging basket installed inside an oven, so that the autoclave can be kept at the desired temperature, while being always kept stirred.

The autoclave is maintained under its autogenous pressure at 150-210°C over a time ranging from 1 day to 7 days, and is then cooled and discharged.

The obtained product is washed with demineralized water, firstly by decantation and then over a filter on which the solid is separated.

This latter is dried for one hour at 120°C and is then calcined for 5 hours at 550°C in air. The exchange into the acidic form is carried out as follows: 10 g of zeolite is suspended in 100 g of a 1% solution of ammonium acetate. The suspension is heated to 50-70°C and is maintained stirred for one hour, then is filtered and the solid is thoroughly washed with demineralized water on the filter.

The operation is repeated three times, and at the end the solid is dried for one hour at 120°C and is then calcined for 5 hours at 550°C in air.

## Examples 1-9

By means of the above reported procedure, 9 preparations were carried out according to the invention.

The composition of the reaction mixture, the operating conditions and the characteristics of the obtained products are reported in Table I.

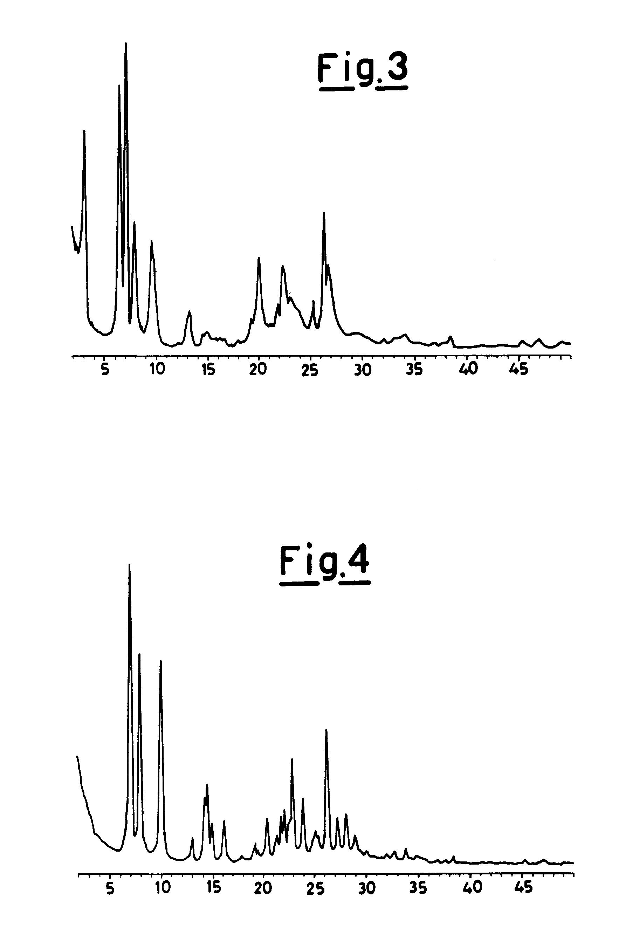

In Figure 3 and Table II, the X-ray diffraction spectrum of the product dried at 120°C, relevant to Example 1, is reported. In Figure 4 and Table III, the X-ray diffraction spectrum of the same product calcined at 550°C is reported. The products of Examples 2 to 9 are characterized by the same X-ray diffraction spectrum as of Example 1.

## Examples 10-15

Still according to the same procedure as above reported, other products were prepared, which do not have the same characteristics as of those as claimed by the present Applicant.

From Table 1, the following can be observed:
- when boron is totally absent, a completely different zeolite, i.e. ZSM-39, is obtained (Example 10);
- when as the nitrogenous base heptamethyleneimine (Example 11), or 1-methyl-piperidine (Example 12) is selected, amorphous products are obtained;
- by starting from a molar ratio of $SiO_2/B_2O_3$ of the reactants equal to 10 (Examples 13), or larger (Example 14), non-zeolitic crystalline products are obtained;
- when the components are reacted in the presence of potassium (Example 15), a different crystalline product is obtained.

Some applications of the herein disclosed material are now given.

## Example 16

Four grams of the material produced according to Example 1 and exchanged into the acidic form according to the processes known from the prior art, is suspended in 120 g of a N/10 solution of CsCl and is kept stirred at room temperature for approximately 10 minutes, after which pH value is measured. To 120 g of a fresh CsCl N/10 solution, a (N/10 and N/100) solution of HCl is added, until the same pH as read in the previous case is obtained.

The dropwise added volume of HCl solution is equivalent to an exchange of $1.5 \times 10^{-1}$ meq/g of Cs.

4

Example 17

0.8 g of zeolite in $H^+$ form prepared as in Example 5 and granulated by pressing into particles having a particle size comprised within the range of from 20 to 40 mesh is charged to a quartz tube and is left in an air stream at 550°C for approximately 15 minutes. Air is then replaced by He and the temperature is reduced down to 450°C. An equimolar mixture of n-hexane and 3-methylpentane diluted in He is then fed, in such a way that the space velocity, expressed as LHSV, is equal to 1. The products leaving the reactor are analysed online by means of a gas-chromatograph.

The conversion is of 30%, and the ratio

$$\frac{\log_{10} (\text{residual n-hexane})}{\log_{10} (\text{residual 3-methylpentane})} = 1$$

Such a ratio is generally referred to as the Constraint Index.

Example 18

100 cc of octene-1 was charged to an autoclave together with 7 g of zeolite powder in the acidic form, prepared as disclosed in Example No. 5.

The suspension was heated to 195°C for 5 hours, with strong stirring. After being cooled, and the catalyst being filtered off, the mixture was analysed by means of gas-chromatography and the chromatographic peaks were identified by mass-spectrometry. The dimers, trimers and tetramers of octene-1 are present:

| | |
|---|---|
| - octene-1 | 4% |
| - dimers $C_{16}H_{32}$ | 50% |
| - trimers $C_{24}H_{48}$ | 32% |
| - tetramers $C_{32}H_{64}$ | 16% |

Table 1

EP 0 293 032 B1

| Composition of the Reaction Mixture | | | | | | | | | Operating Conditions | | | Product Composition | | |
|---|---|---|---|---|---|---|---|---|---|---|---|---|---|---|
| Exam-ple | Silica Source | $SiO_2/B_2O_3$ | R | $R/SiO_2$ | $OH^-/SiO_2$ | $H_2O/SiO_2$ | $SiO_2/M^I_2O_3$ | M | $M_{2/n}O/SiO_2$ | $T^oC$ | pH | Cristallization time (hours) | $SiO_2/B_2O_3$ | $SiO_2/M^I_2O_3$ | Remarks |
| 1 | a | 1.5 | P | 1.00 | 1.40 | 10 | -- | Na | 0.4 | 175 | 11.5 | 72 | 19 | | |
| 2 | a | 1.5 | P | 1.00 | 1.40 | 10 | -- | Na | 0.4 | 175 | 11.5 | 168 | 20 | -- | -- |
| 3 | a | 3 | P | 0.75 | 1.05 | 10 | -- | Na | 0.3 | 175 | 11.4 | 168 | 14 | -- | -- |
| 4 | b | 1.5 | P | 1.00 | 1.40 | 15 | -- | Na | 0.4 | 175 | 10.9 | 168 | 19/5 | -- | -- |
| 5 | a | 2.0 | P | 1.00 | 1.40 | 10 | 50 | Na | 0.4 | 175 | 11.2 | 168 | 135 | 45 | -- |
| 6 | c | 2.0 | P | 0.85 | 1.45 | 15 | 100+ | Na | 0.6 | 175 | 11.3 | 168 | 25 | 131 | -- |
| 7 | c | 1.5 | P | 1.40 | 1.40 | 19 | -- | -- | -- | 175 | 10.7 | 168 | 22 | -- | -- |
| 8 | c | 1.5 | P | 1.00 | 1.60 | 19 | -- | $NH_4$ | 0.6 | 170 | 10.8 | 168 | 2P | -- | -- |
| 9 | c | 1.5 | Hexa | 1.00 | 1.40 | 25 | -- | Na | 0.4 | 170 | 11.1 | 168 | 16.9 | -- | -- |
| 10 | e | --- | P | 0.50 | 0.50 | 30 | -- | -- | -- | 175 | 11.0 | 168 | -- | -- | ZSM 39 |
| 11 | d | 2.0 | Hept | 1.00 | 1.40 | 10 | -- | Na | 0.4 | 170 | 10.8 | 168 | -- | -- | amorphous |
| 12 | d | 1.5 | Mp | 1.00 | 1.40 | 10 | -- | Na | 0.4 | 170 | 10.1 | 168 | -- | -- | amorphous |
| 13 | d | 10 | P | 0.57 | 0.80 | 10 | -- | Na | 0.23 | 170 | 11.3 | 168 | -- | -- | () |
| 14 | d | 20 | P | 0.43 | 0.60 | 10 | -- | Na | 0.17 | 170 | 11.3 | 168 | -- | -- | () |
| 15 | a | 1.5 | P | 1.00 | 1.40 | 10 | -- | K | 0.4 | 175 | 10.7 | 168 | -- | -- | unknown phase |

a = Syloid 404  
b = Ludox HS-40  
c = Aerosil  
d = Syalobloc 47  
e = Tetraethylsilicate

$*M^I_2O_3 = Al_2O_3$

$+M^I_2O_3 = Fe_2O_3$

P = piperidine  
Hexa = hexamethyleneimine  
Hept = Heptamethyleneimine  
Mp = 1-methyl-piperidine

() = Quartz + silica mixture  
+ = unknown crystalline

Table II

| 2 | $\underline{d}$ ($\text{Å} = 10^{-1}\text{nm}$) | 100 $I/I_o$ | 2 | $\underline{d}$ ($\text{Å} = 10^{-1}\text{nm}$) | 100 $I/I_o$ |
|---|---|---|---|---|---|
| 3.28 | 26.94 | 72 | 23.96 | 3.714 | 14 |
| 6.60 | 13.39 | 87 | 25.37 | 3.511 | 18 |
| 7.23 | 12.22 | 100 | 26.41 | 3.374 | 46 |
| 8.00 | 11.05 | 43 | 26.85 | 3.321 | 28 |
| 9.63 | 9.18 | 36 | 29.07 | 3.072 | 7 |
| 9.88 | 8.96 | 31 | 29.68 | 3.009 | 8 |
| 12.93 | 6.85 | 11 | 30.13 | 2.966 | 7 |
| 13.31 | 6.65 | 15 | 32.06 | 2.791 | 6 |
| 14.60 | 6.07 | 7 | 32.85 | 2.727 | 5 |
| 14.99 | 5.91 | 8 | 33.15 | 2.703 | 6 |
| 15.86 | 5.59 | 6 | 34.16 | 2.625 | 7 |
| 16.23 | 5.46 | 6 | 35.65 | 2.518 | 4 |
| 16.59 | 5.34 | 6 | 37.02 | 2.428 | 5 |
| 17.99 | 4.93 | 5 | 37.98 | 2.369 | 5 |
| 19.34 | 4.59 | 12 | 38.47 | 2.340 | 6 |
| 20.09 | 4.42 | 31 | 41.30 | 2.186 | 4 |
| 21.30 | 4.17 | 11 | 45.42 | 1.997 | 5 |
| 21.92 | 4.05 | 17 | 46.92 | 1.936 | 5 |
| 22.41 | 3.967 | 29 | 49.18 | 1.852 | 5 |
| 23.14 | 3.843 | 19 | | | |

Table III

| 2 | $\underline{d}$ ($\text{Å} = 10^{-1}\text{nm}$) | 100 $I/I_o$ | 2 | $\underline{d}$ ($\text{Å} = 10^{-1}\text{nm}$) | 100 $I/I_o$ |
|---|---|---|---|---|---|
| 7.21 | 12.26 | 100 | 28.11 | 3.174 | 19 |
| 8.05 | 10.98 | 71 | 28.99 | 3.080 | 12 |
| 10.12 | 8.74 | 69 | 29.53 | 3.025 | 8 |
| 13.04 | 6.79 | 12 | 30.03 | 2.976 | 8 |
| 14.29 | 6.20 | 25 | 30.67 | 2.915 | 6 |
| 14.48 | 6.12 | 28 | 31.97 | 2.799 | 6 |
| 14.93 | 5.93 | 17 | 32.68 | 2.740 | 7 |
| 16.15 | 5.49 | 17 | 33.80 | 2.652 | 8 |
| 17.90 | 4.95 | 6 | 34.77 | 2.580 | 6 |
| 19.26 | 4.61 | 10 | 35.69 | 2.515 | 5 |
| 19.56 | 4.54 | 8 | 36.86 | 2.439 | 5 |
| 20.44 | 4.34 | 17 | 37.57 | 2.394 | 5 |
| 21.44 | 4.14 | 12 | 38.32 | 2.349 | 6 |
| 21.85 | 4.07 | 18 | 41.17 | 2.192 | 4 |
| 22.13 | 4.02 | 21 | 41.99 | 2.152 | 4 |
| 22.69 | 3.919 | 18 | 43.28 | 2.090 | 4 |
| 22.98 | 3.869 | 37 | 43.85 | 2.065 | 4 |
| 23.99 | 3.709 | 24 | 45.32 | 2.001 | 4 |
| 25.27 | 3.524 | 14 | 46.66 | 1.946 | 4 |
| 25.49 | 3.494 | 13 | 47.00 | 1.933 | 5 |
| 26.30 | 3.389 | 46 | 48.96 | 1.861 | 4 |
| 27.28 | 3.269 | 18 | | | |

**Claims**

**Claims for the following Contracting States : AT, BE, CH, DE, FR, GB, GR, LI, LU, NL, SE**

1. Synthetic, crystalline, porous material of zeolitic nature containing oxides of silicon and boron, characterized in that in the non-calcined state, it complies with the following empirical formula:

$$mM_{2/n}O,pR,xM^I_2O_3,(1\text{-}x)B_2O_3,yM^{II}O_2,qH_2O$$

and in the calcined and anhydrous state, it complies with the following empirical formula:

$$mM_{2/n}O,xM^I_2O_3,(1\text{-}x)B_2O_3,yM^{II}O_2,$$

wherein :

| | |
|---|---|
| M | is an $H^+$, $NH_4^+$ or a metal cation with valence n, |
| $M^I$ | is a trivalent metal selected from Al, Cr, Fe, Ga, V or Mn, |
| $M^{II}$ | is either Si or mixtures thereof with such tetravalent metals as Ge, Ti or Zr, |
| R | is piperidine or hexamethyleneimine, |
| m | has a value comprised within the range of from 0.1 to 1.5, |
| x | has a value comprised within the range of from 0 to 0.9, |
| y | has a value greater than 2 and preferably comprised within the range of from 5 to 300, |
| p | has a value greater than zero and smaller than 3, and |
| q | has a value greater than zero and smaller than 5, |

and that it has a volume of the pores larger than 0.3 $cm^3/g$.

2. Synthetic, crystalline, porous material according to claim 1, wherein the volume of the pores is equal to, or larger than, 0.35 $cm^3/g$.

3. Synthetic, crystalline, porous material according to Claim 1, wherein:
   m is from 0,5 to 1,2;
   x is from nil to 0,2;
   y is from 10 to 100,
   p is from 1 to 3, and
   q is from 1 to 4.

4. Process for hydrothermally preparing a synthetic, crystalline and porous material as defined in Claim 1, comprising the step of reacting, at a temperature of from 130°C to 250°C, at a pH of from 8 to 13, and for a time of from 1 day to 30 days, a silicon derivative, a boron derivative and an organic nitrogenous base selected from piperidine and hexamethyleneimine, with the facultative addition of a derivative of a trivalent metal selected from Al, Cr, Fe, Ga, V and Mn and/or a derivative of a tetravalent metal selected from Ge, Ti, and Zr, and/or a sodium salt or a sodium hydroxide to which other alkali metal or alkaline earth metal salts and/or hydroxides may be added, under the conditions that:
   the molar ratio $SiO_2$ to $B_2O_3$ in the reactants is from 0,5 to 10;
   the molar ratio R to $SiO_2$ in the reactants is from 0,75 to 1,4;
   the molar ratio $M^I_2O_3$ to $SiO_2$ in the reactants is from nil to 0,02;
   the molar ratio $M^{II}O_2$ to $SiO_2$ in the reactants is from 1 to 3, and
   the molar ratio Na to $SiO_2$ in the reactants is from nil to 0,5.

5. Process according to claim 4, wherein the molar ratio of $H_2O/SiO_2$ of the reactants is comprised within the range of from 5 to 50.

6. Process according to claim 4, wherein the derivative of silicon is selected from silica gel, silica sol, aerosil, colloidal silica, sodium silicate or alkyl silicate.

7. Process according to claim 4, wherein the derivative of boron is selected from trialkyl borates, alkali metal borates and ammonium borate, boric acid.

**Claims for the following Contracting State : ES**

1. Process for hydrothermally preparing a synthetic, crystalline and porous material, having in the non calcined state the empirical formula:

$$mM_{2/n}O,pR,xM^I_2O_3,(1-x)B_2O_3,yM^{II}O_2,qH_2O$$

and, in the calcined and anhydrous state, the following empirical formula:

$$mM_{2/n}O,xM^I_2O_3,(1-x)B_2O_3,yM^{II}O_2,$$

wherein :

M      is an $H^+$, $NH_4^+$ or a metal cation with valence n,

$M^I$      is a trivalent metal selected from Al, Cr, Fe, Ga, V or Mn,

$M^{II}$      is either Si or mixtures thereof with such tetravalent metals as Ge, Ti or Zr,

R      is piperidine or hexamethyleneimine,

m      has a value comprised within the range of from 0.1 to 1.5,

x      has a value comprised within the range of from 0 to 0.9,

y      has a value greater than 2 and preferably comprised within the range of from 5 to 300,

p      has a value greater than zero and smaller than 3, and

q      has a value greater than zero and smaller than 5,

and that it has a volume of the pores larger than 0.3 $cm^3/g$.

characterized by comprising the step of reacting, at a temperature of from 130°C to 250°C, at a pH of from 8 to 13, and for a time of from 1 day to 30 days, a silicon derivative, a boron derivative and an organic nitrogenous base selected from piperidine and hexamethyleneimine, with the facultative addition of a derivative of a trivalent metal selected from Al, Cr, Fe, Ga, V and Mn and/or a derivative of a tetravalent metal selected from Ge, Ti , and Zr, and/or a sodium salt or a sodium hydroxide to which other alkali metal or alkaline earth metal salts and/or hydroxides may be added, under the conditions that:

the molar ratio $SiO_2$ to $B_2O_3$ in the reactants is from 0,5 to 10;

the molar ratio R to $SiO_2$ in the reactants is from 0,75 to 1,4;

the molar ratio $M^I_2O_3$ to $SiO_2$ in the reactants is from nil to 0,02;

the molar ratio $M^{II}O_2$ to $SiO_2$ in the reactants is from 1 to 3, and

the molar ratio Na to $SiO_2$ in the reactants is from nil to 0,5.

2. Process according to Claim 1 , characterized in that the molar ratio $H_2O$ to $SiO_2$ in the reactants is from 5 to 50.

3. Process according to Claim 1 , characterized in that the derivative of silicon is selected from silica gel,, silica sol, colloidal silica, sodium silicate and alkyl silicate.

4. Process according to Claim 1, characterized in that the derivative of boron is selected from trialkyl borates, alkali metal borates, ammonium borate and boric acid.

**Patentansprüche**
**Patentansprüche für folgende Vertragsstaaten : AT, BE, CH, DE, FR, GB, GR, LI, LU, NL, SE**

1. Synthetisches, Kristallines, poröses Material von Zeolith-Natur Mit einem Gehalt an Silicium- und Boroxiden, dadurch gekennzeichnet, daß es im nicht-calcinierten Zustand der folgenden empirischen Formel:

$$mM_{2/n}O,pR,xM^I_2O_3,(1-x)B_2O_3,yM^{II}O_2,qH_2O$$

und im calcinierten und wasserfrein Zustand der folgenden empirischen Formel:

$$mM_{2/n}O,xM^I_2O_3,(1-x)B_2O_3,yM^{II}O_2$$

entspricht, worin:

M  für ein $H^+$-, $NH_4^+$- oder ein Metallkation mit der Wertigkeit n steht,

$M^I$  ein dreiwertiges, unter Al, Cr, Fe, Ga, V oder Mn ausgewähltes Metall bedeutet,

$M^{II}$  entweder Silicium bedeutet oder für Gemische hievon mit solchen vierwertigen Metallen wie Ge, Ti oder Zr steht,

R  Piperidin oder Hexamethylenimin darstellt,

m  einen Wert im Bereich von 0,1 bis 1,5 aufweist,

x  einen Wert im Bereich von 0 bis 0,9 hat,

y  einen Wert größer als 2 und vorzugsweise im Bereich von 5 bis 300 hat,

p  einen Wert größer als Null und kleiner als 3 aufweist und

q  einen Wert größer Null und kleiner als 5 hat,

und daß es ein Porenvolumen von mehr als 0,3 $cm^3$/g aufweist.

2.  Synthetisches, kristallines, poröses Material nach Anspruch 1, worin das Porenvolumen gleich oder größer als 0,35 $cm^3$/g ist.

3.  Synthetisches, kristallines, poröses Material nach Anspruch 1, worin:

m  von 0,5 bis 1,2 beträgt;

x  von 0 bis 0,2 beträgt;

y  von 10 bis 100 beträgt;

p  von 1 bis 3 beträgt und

q  von 1 bis 4 beträgt.

4.  Verfahren zur hydrothermalen Herstellung eines synthetischen, kristallinen und porösen Materials, wie in Anspruch 1 definiert, umfassend die Stufe des Umsetzens eines Siliciumderivats, eines Borderivats und einer organischen stickstoffhältigen Base, ausgewählt unter Piperidin und Hexamethylenimin, unter fakultativer Zugabe eines Derivats eines dreiwertigen Metalles, ausgewählt unter Al, Cr, Fe, Ga, V und Mn, und/oder eines Derivats eines vierwertigen Metalles, ausgewählt unter Ge, Ti und Zr, und/oder eines Natriumsalzes oder eines Natriumhydroxids, zu dem andere Alkalimetall- oder Erdalkalimetallsalze und/oder -hydroxide zugesetzt werden können, bei einer Temperatur von 130°C bis 250°C, einem pH-Wert von 8 bis 13 und während einer Zeit von 1 Tag bis 30 Tagen, unter solchen Bedingungen, daß:

das Molverhältnis $SiO_2$ zu $B_2O_3$ in den Reaktanten von 0,5 bis 10 beträgt;

das Molverhältnis R zu $SiO_2$ in den Reaktanten von 0,75 bis 1,4 beträgt;

das Molverhältnis $M^I_2O_3$ zu $SiO_2$ in den Reaktanten von 0 bis 0,02 beträgt;

das Molverhältnis $M^{II}O_2$ zu $SiO_2$ in den Reaktanten von 1 bis 3 beträgt und

das Molverhältnis Na zu $SiO_2$ in den Reaktanten von 0 bis 0,5 beträgt.

5.  Verfahren nach Anspruch 4, worin das Molverhältnis von $H_2O/SiO_2$ der Reaktanten im Bereich von 5 bis 50 liegt.

6.  Verfahren nach Anspruch 4, worin das Siliciumderivat unter Silicagel, Silicasol, Aerosil, kolloidaler Kieselsäure, Natriumsilicat oder Alkylsilicat ausgewählt ist.

7.  Verfahren nach Anspruch 4, worin das Borderivat unter Trialkylboraten, Alkalimetallboraten und Ammoniumborat sowie unter Borsäure ausgewählt ist.

**Patentansprüche für folgenden Vertragsstaat : ES**

1.  Verfahren zum hydrothermalen Herstellen eines synthetischen, kristallinen und porösen Materials, das im nicht-calcinierten Zustand die empirische Formel:

$mM_{2/n}O, pR, xM^I_2O_3, (1-x)B_2O_3, yM^{II}O_2, qH_2O$

und im calcinierten und wasserfrein Zustand die folgende empirische Formel:

$mM_{2/n}O, xM^I_2O_3, (1-x)B_2O_3, yM^{II}O_2$

aufweist, worin:

M      für ein $H^+$-, $NH_4^+$- oder ein Metallkation mit der Wertigkeit n steht,

$M^I$    ein dreiwertiges, unter Al, Cr, Fe, Ga, V oder Mn ausgewähltes Metall bedeutet,

$M^{II}$   entweder Silicium bedeutet oder für Gemische hievon mit solchen vierwertigen Metallen wie Ge, Ti oder Zr steht,

R      Piperidin oder Hexamethylenimin darstellt,

m     einen Wert im Bereich von 0,1 bis 1,5 aufweist,

x      einen Wert im Bereich von 0 bis 0,9 hat,

y      einen Wert größer als 2 und vorzugsweise im Bereich von 5 bis 300 hat,

p      einen Wert größer als Null und kleiner als 3 aufweist und

q      einen Wert größer Null und kleiner als 5 hat,

und das ein Porenvolumen von mehr als 0,3 $cm^3/g$ aufweist, dadurch gekennzeichnet, daß es die Stufe des Umsetzens eines Siliciumderivats, eines Borderivats und einer organischen stickstoffhältigen Base, ausgewählt unter Piperidin und Hexamethylenimin, unter fakultativer Zugabe eines Derivats eines dreiwertigen Metalles, ausgewählt unter Al, Cr, Fe, Ga, V und Mn, und/oder eines Derivats eines vierwertigen Metalles, ausgewählt unter Ge, Ti und Zr, und/oder eines Natriumsalzes oder eines Natriumhydroxids, zu dem andere Alkalimetall- oder Erdalkalimetallsalze und/oder -hydroxide zugesetzt werden können, bei einer Temperatur von 130°C bis 250°C, einem pH-Wert von 8 bis 13 und während einer Zeit von 1 Tag bis 30 Tagen, unter solchen Bedingungen umfaßt, daß:

das Molverhältnis $SiO_2$ zu $B_2O_3$ in den Reaktanten von 0,5 bis 10 beträgt;

das Molverhältnis R zu $SiO_2$ in den Reaktanten von 0,75 bis 1,4 beträgt;

das Molverhältnis $M^I_2O_3$ zu $SiO_2$ in den Reaktanten von 0 bis 0,02 beträgt;

das Molverhältnis $M^{II}O_2$ zu $SiO_2$ in den Reaktanten von 1 bis 3 beträgt und

das Molverhältnis Na zu $SiO_2$ in den Reaktanten von 0 bis 0,5 beträgt.

**2.** Verfahren nach Anspruch 1, dadurch gekennzeichnet, daß das Molverhältnis von $H_2O/SiO_2$ der Reaktanten im Bereich von 5 bis 50 liegt.

**3.** Verfahren nach Anspruch 1, dadurch gekennzeichnet, daß das Siliciumderivat unter Silicagel, Silicasol, Aerosil, kolloidaler Kieselsäure, Natriumsilicat oder Alkylsilicat ausgewält ist.

**4.** Verfahren nach Anspruch 1, dadurch gekennzeichnet, daß das Borderivat unter Trialkylboraten, Alkalimetallboraten und Ammoniumborat sowie unter Borsäure ausgewählt ist.

## Revendications
### Revendications pour les Etats contractants suivants : AT, BE, CH, DE, FR, GB, GR, LI, LU, NL, SE

**1.** Matériau synthétique, cristallin et poreux, de nature zéolithique, contenant des oxydes de silicium et de bore, caractérisé en ce qu'il correspond, à l'état non calciné, à la formule empirique suivante :

$$mM_{2/n}O,pR,xM^I_2O_3,(1-x)B_2O_3,yM^{II}O_2,qH_2O$$

et à l'état calciné et anhydre, à la formule empirique suivante :

$$mM_{2/n}O,xM^I_2O_3,(1-x)B_2O_3,yM^{II}O_2$$

formules dans lesquelles :

M représente $H^+$, $NH_4^+$, ou un cation métallique de valence n,

$M^I$ représente un métal trivalent choisi parmi Al, Cr, Fe, Ga, V et Mn,

$M^{II}$ représente soit Si, soit un mélange de celui-ci avec des métaux tétravalents tels que Ge, Ti ou Zr,

R représente la pipéridine ou l'hexaméthylène-imine,

m possède une valeur située dans l'intervalle allant de 0,1 à 1,5,

x possède une valeur située dans l'intervalle allant de 0 à 0,9,

y possède une valeur supérieure à 2 et de préférence située dans l'intervalle allant de 5 à 300,

p possède une valeur supérieure à 0 et inférieure à 3, et

g possède une valeur supérieure à 0 et inférieure à 5,

et en ce qu'il présente un volume de pores supérieur à 0,3 $cm^3/g$.

**2.** Matériau synthétique, cristallin et poreux, conforme à la revendication 1, dans lequel le volume de pores est égal ou supérieur à 0,35 $cm^3$/g.

**3.** Matériau synthétique, cristallin et poreux, conforme à la revendication 1, dans lequel
m vaut de 0,5 à 1,2,
x vaut de 0 à 0,2,
y vaut de 10 à 100,
p vaut de 1 à 3, et
q vaut de 1 à 4.

**4.** Procédé de préparation, par voie hydrothermique, d'un matériau synthétique, cristallin et poreux, tel que défini dans la revendication 1, ledit procédé comprenant l'étape consistant à faire réagir, à une température de 130°C à 250°C, à un pH de 8 à 13 et pendant un laps de temps de 1 jour à 30 jours, un dérivé du silicium, un dérivé du bore et une base organique azotée choisie parmi la pipéridine et l'hexaméthylène-imine, en ajoutant facultativement un dérivé d'un métal trivalent choisi parmi Al, Cr, Fe, Ga, V et Mn, et/ou un dérivé d'un métal tétravalent choisi parmi Ge, Ti et Zr, et/où un sel de sodium ou de l'hydroxyde de sodium, auquel on peut ajouter d'autres sels et/ou hydroxydes de métal alcalin ou de métal alcalino-terreux, sous réserve que :
le rapport molaire $SiO_2/B_2O_3$ des réactifs vaille de 0,5 à 10,
le rapport molaire $R/SiO_2$ des réactifs vaille de 0,75 à 1,4,
le rapport molaire $M^I_2O_3/SiO_2$ des réactifs vaille de 0 à 0,02,
le rapport molaire $M^{II}O_2/SiO_2$ des réactifs vaille de 1 à 3, et
le rapport molaire $Na/SiO_2$ des réactifs vaille de 0 à 0,5.

**5.** Procédé conforme à la revendication 4, dans lequel le rapport molaire $H_2O/SiO_2$ des réactifs se situe dans l'intervalle allant de 5 à 50.

**6.** Procédé conforme à la revendication 4, dans lequel le dérivé du silicium est choisi parmi un gel de silice, un sol de silice, une silice fumée, une silice colloïdale, un silicate de sodium et un silicate d'alkyle.

**7.** Procédé conforme à la revendication 4, dans lequel le dérivé du bore est choisi parmi les borates de trialkyle, les borates de métal alcalin, le borate d'ammonium et l'acide borique.

**Revendications pour l'Etat contractant suivant : ES**

**1.** Procédé de préparation, par voie hydrothermique, d'un matériau synthétique, cristallin et poreux, correspondant, à l'état non calciné, à la formule empirique suivante :

$$mM_{2/n}O,pR,xM^I_2O_3,(1-x)B_2O_3,yM^{II}O_2,qH_2O$$

et à l'état calciné et anhydre, à la formule empirique suivante :

$$mM_{2/n}O,xM^I_2O_3,(1-x)B_2O_3,yM^{II}O_2$$

formules dans lesquelles :
M représente $H^+$, $NH_4^+$, ou un cation métallique de valence n,
$M^I$ représente un métal trivalent choisi parmi Al, Cr, Fe, Ga, V et Mn,
$M^{II}$ représente soit Si, soit un mélange de celui-ci avec des métaux tétravalents tels que Ge, Ti ou Zr,
R représente la pipéridine ou l'hexaméthylène-imine,
m possède une valeur située dans l'intervalle allant de 0,1 à 1,5,
x possède une valeur située dans l'intervalle allant de 0 à 0,9,
y possède une valeur supérieure à 2 et de préférence située dans l'intervalle allant de 5 à 300,
p possède une valeur supérieure à 0 et inférieure à 3, et
g possède une valeur supérieure à 0 et inférieure à 5,
et présentant un volume de pores supérieur à 0,3 $cm^3$/g,
ledit procédé étant caractérisé en ce qu'il comprend l'étape consistant à faire réagir, à une température de 130°C à 250°C, à un pH de 8 à 13 et pendant un laps de temps de 1 jour à 30 jours, un dérivé du silicium, un dérivé du bore et une base organique azotée choisie parmi la pipéridine et l'hexaméthylène-imine, en ajoutant facultativement un dérivé d'un métal trivalent choisi parmi Al, Cr, Fe, Ga, V et Mn, et/ou un dérivé d'un métal tétravalent choisi parmi Ge, Ti et Zr, et/ou un sel de sodium ou de l'hydroxyde de sodium, auquel on peut ajouter d'autres sels et/ou hydroxydes de métal alcalin ou de métal alcalino-terreux, sous réserve que :

le rapport molaire $SiO_2/B_2O_3$ des réactifs vaille de 0,5 à 10,
le rapport molaire $R/SiO_2$ des réactifs vaille de 0,75 à 1,4,
le rapport molaire $M^I_2O_3/SiO_2$ des réactifs vaille de 0 à 0,02,
le rapport molaire $M^{II}O_2/SiO_2$ des réactifs vaille de 1 à 3, et
le rapport molaire $Na/SiO_2$ des réactifs vaille de 0 à 0,5.

2. Procédé conforme à la revendication 1, caractérisé en ce que le rapport molaire $H_2O/SiO_2$ des réactifs se situe dans l'intervalle allant de 5 à 50.

3. Procédé conforme à la revendication 1, caractérisé en ce que le dérivé du silicium est choisi parmi un gel de silice, un sol de silice, une silice fumée, une silice colloïdale, un silicate de sodium et un silicate d'alkyle.

4. Procédé conforme à la revendication 1, caractérisé en ce que le dérivé du bore est choisi parmi les borates de trialkyle, les borates de métal alcalin, le borate d'ammonium et l'acide borique.

## Fig.1

## Fig.2

Fig.3

Fig.4